(19) Europäisches Patentamt
European Patent Office
Office européen des brevets

(11) EP 1 968 916 B1

(12) **EUROPEAN PATENT SPECIFICATION**

(45) Date of publication and mention of the grant of the patent:
**11.07.2012 Bulletin 2012/28**

(21) Application number: **06820715.8**

(22) Date of filing: **30.11.2006**

(51) Int Cl.:
*C07D 307/935* (2006.01) *C07B 57/00* (2006.01)

(86) International application number:
**PCT/HU2006/000105**

(87) International publication number:
**WO 2007/066160 (14.06.2007 Gazette 2007/24)**

(54) **Process for the separation of optical isomers of the corey lactone**

Verfahren zur Trennung optischer Isomere des Corey-Lactons

Procédé pour la séparation d'isomères optiques de lactone de corey

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **09.12.2005 HU 0501144**

(43) Date of publication of application:
**17.09.2008 Bulletin 2008/38**

(73) Proprietor: **Chinoin Gyógyszer és Vegyészeti Termékek Gyára Zrt.**
**1045 Budapest (HU)**

(72) Inventors:
- **SZÁNYA, Tibor**
  **8200 Veszprém (HU)**
- **HANÁK, László**
  **8200 Veszprém (HU)**
- **KOZMA, Gábor**
  **1156 Budapest (HU)**
- **NÉMETH, Attila**
  **2131 Göd (HU)**
- **FÓNAGY, László**
  **1174 Budapest (HU)**
- **KARDOS, Zsuzsanna**
  **1132 Budapest (HU)**
- **SZABÓ, Tibor**
  **1133 Budapest (HU)**
- **VAJDA, Ervin**
  **1174 Budapest (HU)**
- **HORVÁTH, Géza**
  **8200 Veszprém (HU)**
- **SZABÓNÉ RAVASZ, Bernadett**
  **8200 Veszprém (HU)**
- **STRBKA, Andrásné**
  **8200 Veszprém (HU)**
- **NAGY, Melinda**
  **8200 Veszprém (HU)**
- **MOLNÁR, Zoltán**
  **8861 Szepetnek (HU)**

(74) Representative: **Ori, Janos et al**
**Chinoin Gyogyszer és Vegyészeti Termékek Gyara Zrt.**
**To u. 1-5.**
**H-1045 Budapest (HU)**

(56) References cited:
**EP-A1- 0 501 310 US-A1- 2002 095 061**

- **ABOUL-ENEIN, H. Y. ET AL.: "Enantiomeric resolution of some substituted tetraolone derivatives on amylose tris(3,5-dimethylphenylcarbamate) chiral stationary phase in reversed phase mode", JOURNAL OF SEPARATION SCIENCE, vol. 24, no. 10-11, 16 November 2001 (2001-11-16), pages 831-834, DOI: 10.1002/1615-9314(20011101)24:10/11<831::AID-JSSC831>3.0.CO;2-L**
- **BONATO, S. P. ET AL.: "Enantioselective HPLC analysis of propafenone and of its main metabolites using polysaccharide and protein-based chiral stationary phases", BIOMEDICAL CHROMATOGRAPHY, vol. 14, no. 4, 5 June 2000 (2000-06-05), pages 227-233, DOI: 10.1002/1099-0801(200006)14:4<227::AID-BMC971>3.0.CO;2-Q**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **ROUSSEL, C. ET AL.: "Chiral separation of some 4a-methyl-1,2,3,4,4a,9a-hexahydro-fluoren- 9-one derivatives as a probe for difference in solvation by 2-propanol of carbamate moiety in chiralcel OD-H, chiralpak AD, and chiralpak AS chiral stationary phases", CHIRALITY, vol. 10, no. 8, 6 January 1999 (1999-01-06), pages 770-777, DOI: 10.1002/(SICI)1520-636X(1998)10:8<770:: AID -CHIR8>3.0.CO;2-0**

## Description

The subject of the present invention is the separation of the optical isomers of the racemic hexahydro-5-hydroxy-4-(hydroxymethyl)-2*H*-cyclopenta[b]furan-2-one ("Corey-lactone") of formula (I).

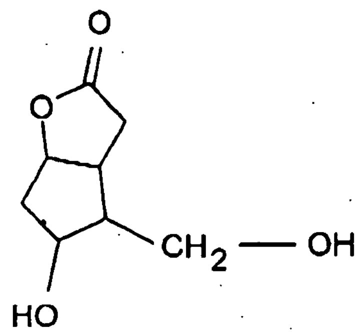

(I)

by liquid chromatography. The laevo-rotatory (-) isomer of the compound of formula (I) is a valuable prostaglandin intermediate.

The optical isomers of the compound of formula (I) were prepared by enzymatic resolution (EP-501310 A).

The enzymatic resolution method requires long reaction time, and in addition, the regeneration of the enzyme in industrial scale is difficult.

Hence, there was a substantial need to develop an effective method to ensure also in industrial scale the preparation of the isomers of the compound of formula (I), first of all the laevo-rotatory optical isomer (-)(I), while retaining the high optical purity and the good yield.

Surprisingly, we have found that the optical isomers of the racemic compound of formula (I) can be separated by a liquid chromatographic method and this method ensures the preparation of the laevo-rotatory (-) optical isomer in good yield and high purity, because the (+) (I) compound leaves together with the impurity.

Thus the racemic compound of formula (I), or the mixture of the optical isomers of the compound of formula (I) of ratio off 1:1, is subjected to liquid chromatographic resolution where during the said process for chiral stationary phase amylose-tris [(S)-α-methylbenzylcarbamate] or amylose-tris-3,5-dimethylphenylcarbamate and as for mobile phase methanol - normal hexane mixtures or methanol-acetonitrile mixtures or methanol-isopropyl alcohol mixtures or isopropylalcohol-n-hexane-methanol mixtures or isopropylalcohol-n-hexane-ethanol mixtures or methanol alone or acetonitrile alone are applied and if desired, the resulted dextro- and laevo-rotatory optical isomers are isolated.

The chiral stationary phase is coated to silicagel, cheramic or to other usual support which are amylose-tris-[(S)-α-methylbenzylcarbamate] or amylose-tris-3,5-dimethylphenylcarbamate the corresponding commercial products are for example Chiralpak®-AS or Chiralpak®-AD or Chiralpak®-IA (Supplier: Daicel Chem. Ind. Ltd.).

As mobile phase for example the methanol - normal hexane - isopropyl alcohol mixtures or methanol -isopropyl alcohol mixtures or isopropyl alcohol - normal hexane mixtures or ethanol - normal hexane mixtures are applied. Methanol-acetonitrile mixtures are well applicable. Furthermore acetonitrile or methanol alone are also applicable.

The resolution is usually performed at a temperature between 10-40 C°, by batch or continuous process. The applied pressure is usually below 50 bar.

The process according to the invention can be carried out by batch or continuous process. The continuous process can be performed by various methods, one of them is the simulated moving bed (SMB) liquid chromatogaphy.

The SMB method is described for example in the patent application of publication number: EP-719742A2.

Further details of the invention are demonstrated in the following examples, without limiting the claims of Applicant to the examples.

Figure 1 shows the HPLC results in Example 1.
Figure 2 shows the drawing of equipment used in Example 2.

## Examples

<u>Example 1a</u>

In a preparative chromatography column ($D_b$=2 cm, L= 50 cm) 92.5 g of Chiralpak®-AD packing (amylose-3,5-dimethylphenyl carbamate coated to silicagel) of particle size 20 μm is filled during 60 minutes using vibrational method.

The column is equilibrized with the mixture of 75 volume/volume % acetonitrile-25 volume/volume % methanol

at 20 ˚C, then with a volumetric velocity of 5 $cm^3$/min 110 $cm^3$ of the above eluent solvent containing 10.56 g compound/$dm^3$ eluent (5.3 g (+) (I), 4.3 g (-) (I) and 0.96 g Imp) is added. The column is then washed with the pure eluent of the above solvent composition. The samples taken at the end of the column are analysed with HPLC. Results are summarized in Table I. and graphically in Figure 1.

Table I

| | | | +(I) g/$dm^3$ | -(I) g/$dm^3$ | Imp g/$dm^3$ |
|---|---|---|---|---|---|
| Sample No | Σ volume($cm^3$) | input | 5.0043 | 3.86 | 0.95 |
| 5 | 100.78 | | | | 1.12 |
| 7 | 109.28 | | 0.0000 | | 2.19 |
| 9 | 117.66 | | 0.6527 | 0.00 | 1.51 |
| 11 | 126.15 | | 5.3089 | 0.05 | 1.06 |
| 13 | 134.54 | | 5.7941 | 0.05 | 1.06 |
| 15 | 143.06 | | 5.7185 | 0.17 | 1.02 |
| 17 | 151.50 | | 5.5241 | 1.78 | 1.01 |
| 19 | 160.08 | | 5.2392 | 4.01 | 1.01 |
| 23 | 177.10 | | 5.2725 | 4.28 | 0.96 |
| 27 | 194.35 | | 5.1037 | 4.14 | 0.58 |
| 31 | 211.27 | | 4.0542 | 3.88 | 0.20 |
| 32 | 215.50 | | 2.7495 | 3.60 | 0.12 |
| 33 | 219.79 | | 1.5130 | 3.53 | 0.15 |
| 34 | 224.11 | | 0.5456 | 3.39 | 0.15 |
| 35 | 228.20 | | 0.2368 | 3.79 | 0.22 |
| 37 | 236.48 | | 0.0724 | 3.50 | 0.18 |
| 39 | 244.92 | | 0.0884 | 3.01 | 0.20 |
| 41 | 253.20 | | 0.1268 | 1.96 | 0.20 |
| 43 | 261.47 | | 0.0954 | 1.12 | 0.19 |
| 45 | 269.94 | | 0.0958 | 0.61 | 0.21 |
| 47 | 278.36 | | 0.1488 | 0.30 | 0.21 |
| 49 | 286.57 | | 0.1071 | 0.10 | 0.21 |
| 51 | 294.87 | | 0.1096 | 0.03 | 0.19 |
| 53 | 303.16 | | 0.2014 | 0.00 | 0.22 |
| 55 | 311.42 | | 0.0853 | 0.00 | 0.22 |
| Imp = impurity | | | | | |

In Figure 1 on the horizontal axis the amount of the pure eluent, whereas on the vertical axis the concentrations of compound (+)(I), (-)(I) and of the impurity present in the eluent are shown.

The results clearly indicate that the (-)(I) compound can well and in pure form separated from the (+)(I) compound, the latter leaving the system together with the impurity. The selectivity value [(-)(I)/(+)(I)] for the (-)(I) compound is (α) = 2.5.

The selectivity values (α) obtained at 20 ˚C for the various chiral stationary phases and mobile phases applied in the method according to the invention are summarized in the following tables:

Table II.

$$\alpha = \frac{\text{compound of formula (-)(I)}}{\text{compound of formula (+)(I)}}$$

(continued)

| Chiral stationary phase | Mobile phase V/V % | α |
|---|---|---|
| Chiralpak®AD | methanol 100 | 1.57 |
| " | methanol 80<br>n-hexane 20 | 1.69 |
| " | methanol 80<br>acetonitrile 20 | 1.80 |
| " | methanol 70<br>acetonitrile 30 | 2.10 |
| " | methanol 50<br>acetonitrile 50 | 2.37 |
| Chiral stationary phase | Mobile phase V/V% | α |
| Chiralpak® AD | methanol 25<br>acetonitrile 75 | 2.50 |
| Chiralpak® IA | methanol 25<br>acetonitrile 75 | 1.34 |

Table III.

| $\alpha = \frac{\text{compound of formula (+)(I)}}{\text{compound of formula (-)(I)}}$ | | |
|---|---|---|
| Chiralpak®AS | methanol 50<br>Isopropyl alcohol 50 | 2.13 |
| " | methanol 100 | 1.43 |
| " | Isopropylalcohol 40<br>n-hexane 40<br>metanol 20 | 1.52 |
| Chiralpak®AS | ethanol 40<br>n-hexane 40<br>Isopropyl alcohol 20 | 1.37 |

Example 2

Resolution of the racemic hexahydro-5-hydroxy-4-(hydroxymethyl)-2*H*-cyclopenta-[*b*]furan-2-one compound of formula (I) by SMB method

In a small scale laboratory SMB equipment with 1:1:2:0 column configuration and open eluent circulation (Prepared by: University of Veszprém - Központi Gépmühely) 4 columns (inner diameter $D_b$ = 1 cm, column length L = 25 cm) are filled, dry, with Chiralpack®-AD chiral stationary phase, in a period of 60-60 minutes, by vibrational method. The schematic drawing of the apparatus is shown on Figure 2.

| | **Filling amount** | **Particle size** |
|---|---|---|
| Column I. | 12.79 g | 20 micrometer |
| Column II | 12.07 g | 20 micrometer. |
| Column III | 12.96 g | 20 micrometer |

(continued)

| | Filling amount | Particle size |
|---|---|---|
| Column IV | 12.84 g | 20 micrometer |

To the SMB apparatus the solvent mixture of composition 75:25 volume/volume% acetonitrile-methanol is added. The columns I-IV are equilibrized at 20 C° with this solvent mixture. In the separation process the racemic compound of formula (I) was dissolved in the solvent mixture of composition 75:25 volume/volume% acetonitrile-methanol, in a concentration of 42.2 g/dm$^3$ [21.2 g (+)(I), 17.2 g (-)(I), 3.8 g Imp].

During the separation the switching time and the volumetric velocity was changed (D, E, F, LROUT)

The meanings of the applied denotions are as follows:

| | |
|---|---|
| P | pressure at the inlet point of the fresh eluent (bar) |
| E | volumetric velocity (cm$^3$/inin) of the output extract. |
| LROUT R | volume speed (cm$^3$/min) of the raffinatum leaving the SMB equipment, in case of column configuration 1: 1:2:0. |
| F | inlet volumetric velocity (cm$^3$/min) of the mixture to be purified /racemic (I) of 42 g/cm$^3$ concentration in (75:25 %v/v) acetonitrile-methanol mixture [21.2 g (+)(I), 17.2 g (-)(I), 3.8 g Imp]/, |
| T | switching time (min) |
| D | volumetric velocity (cm$^3$/min) of the fresh eluent (75:25 %v/v) acetonitrile-methanol |

| F = 1.8 cm$^3$/min T = 2 min | | | | | |
|---|---|---|---|---|---|
| Sample | P (bar) | E (cm$^3$/min) | LROUT (cm$^3$/min) | D (cm$^3$/min) | F (cm$^3$/min) |
| E I-II | 30 | 8.53 | 9.16 | 15.88 | 1.81 |
| E III | 30 | 6.75 | 7.19 | 15.75 | 1.79 |

Summary of the important operational characteristics:

| | |
|---|---|
| Pressure: | 30-35 bar, maximum pressure 50 bar |
| Eluent: | 75:25 % v/v acetonitrile-methanol |
| D = | 15.75 -15.88 cm$^3$/min |
| E = | 8.53 - 6.75 cm$^3$/mn |
| F = | 1.81 - 1.79 cm$^3$/min |
| LROUT = | 9.16 - 7.19 cm$^3$/min |
| Switching time: | 2 min |
| Extract: | 88.62 - 93.12 area/area% (-)(I) compound 11.38 - 6.88 area/area% (+)(I) compound 0 area/area% Imp (Impurity) |
| LROUT | 81.5 - 82 area/area% (+)(I) compound 1.8 - 2 area/area% (-)(I) compound 16.7 - 16 area/area% Imp (impurity) |

Evaporation of the extracts and crystallisation under cooling:

Of the obtained fractions, the extracts E I-III (summa volume 198.35 cm$^3$) were evaporated on a Rotadest apparatus in weak vacuum at 75-80 C° to a volume of 7.22 cm$^3$ and then concentrate was then cooled in freezer at (-) 15 - (-)20 C° for 6 hours. The crystals were separated from the mother liquor, washed with 1.05 g of hexane and dried under air, at room temperature.

Mass of the crystals: 0.42 g, purity: 99.07 % m/m (-)(I) compound. Yield of the crystallisation calculated for the (-)(I) compound: 84.30 %.

Chinoin SMB-4/B

| | |
|---|---|
| Based on E I-III | |
| Tact time: | 2 min |

(continued)

| | | |
|---|---|---|
| Based on E I-III | | |
| Cycle number: | | 3 |
| Operational time: | | 4x2x3 = 24 min |
| Yield of the crystals: | | 420 mg (2. sample) |
| | purity: | 99.07 % m/m |
| Productivity (SMB + crystallisation): | | |

$$\frac{420\ \text{mg} \times 0.9907}{24\ \text{min} \times 51.26\ \text{g filling}} = 0.3382\ \frac{\text{mg (-)(I) compound}}{\text{g filling min}} =$$

$$= 0.487\ \frac{\text{kg (-)(I) compound}}{\text{kg filling day}}$$

Specific solvent consumption (SMB+crystallisation): 0.92 $m^3$ fresh eluent/kg (-)(I) compound

SMB (-)(I) compound yield: 97.9 %
SMB + crystallisation (-)(I) compound yield: 84.3 %

## Claims

1. Process for the separation of the optical isomers of the compound

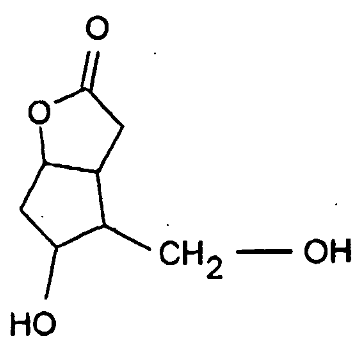

(I)

hexahydro-4-(hydroxymethyl)-5-hydroxy-2*H*-cyclopenta[b]furan-2-one of formula (I), **characterized in that**, the racemic compound of formula (I), or the mixture of the optical isomers of the compound of formula (I) of ratio off 1:1, is subjected to liquid chromatographic resolution where during the said process for chiral stationary phase amylose-tris [(S)-α-methylbenzylcarbamate] or amylose-tris-3,5-dimethylphenylcarbamate and as for mobile phase methanol - normal hexane mixtures or methanol-acetonitrile mixtures or methanol-isopropyl alcohol mixtures or isopropylalcohol-n-hexane-methanol mixtures or isopropylalcohol-n-hexane-ethanol mixtures or methanol alone or acetonitrile alone are applied and if desired, the resulted dextro- and laevo-rotatory optical isomers are isolated.

2. The processes as defined in Claim 1, **characterized in that**, as for chiral stationary phase amylose-tris-3,5-dimethylphenylcarbamate and as for mobile phase mixtures of acetonitrile and methanol are used.

3. The process as defined in Claim 1, **characterized in that**, as for chiral stationary phase amylose tris-[(S)-α-methylbenzylcarbamate] and as for mobile phase isopropyl alcohol - normal hexane mixtures or isopropyl alcohol -normal hexane - methanol mixtures are used.

4. The process as defined in Claim1, **characterized in that**, continuous chromatographic process is used.

5. The process as defined in Claim 4, **characterized in that**, simulated moving bed (= SMB) liquid chromatographic method is used.

6. The process as defined in Claim 1, **characterized in that**, batch chromatographic process is used.

7. The process as defined in Claim 1, **characterized in that**, the laevo-rotatory and dextro-rotatory optical isomers are isolated by applying evaporation and crystallisation methods known per se.

8. The process as defined in Claim 1, **characterized in that**, the liquid chromatographic resolution is performed at a temperature between + 10 C° and + 40C°.

## Patentansprüche

1. Verfahren zur Trennung der optischen Isomere der Verbindung

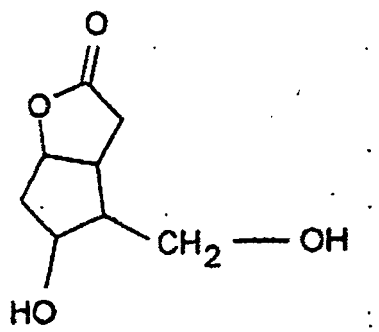

(I)

Hexahydro-4-(hydroxymethyl)-5-hydroxy-2H-cyclopenta[b]furan-2-on der Formel (I), **dadurch gekennzeichnet, dass** man die racemische Verbindung der Formel (I) oder das Gemisch der optischen Isomere der Verbindung der Formel (I) im Verhältnis von 1:1 einer flüssigkeitschromatographischen Trennung unterwirft, wobei man bei dem Verfahren als chirale stationäre Phase Amylosetris[(S)-α-Methylbenzylcarbamat] oder Amylosetris-3,5-dimethylphenylcarbamat und als mobile Phase Mischungen von Methanol und n-Hexan oder Mischungen von Methanol und Acetonitril oder Mischungen von Methanol und Isopropylalkohol oder Mischungen von Isopropylalkohol, n-Hexan und Methanol oder Mischungen von Isopropylalkohol, n-Hexan und Ethanol oder Methanol alleine oder Acetonitril alleine verwendet und gegebenenfalls die resultierenden rechts-und linksdrehenden optischen Isomere isoliert.

2. Verfahren nach Anspruch 1, **dadurch gekennzeichnet, dass** man als chirale stationäre Phase Amylosetris-3,5-dimethylphenylcarbamat und als mobile Phase Mischungen von Acetonitril und Methanol verwendet.

3. Verfahren nach Anspruch 1, **dadurch gekennzeichnet, dass** als chirale stationäre Phase Amylosetris[(S)-α-Methylbenzylcarbamat] und als mobile Phase Mischungen von Isopropylalkohol und n-Hexan oder Isopropylalkohol, n-Hexan und Methanol verwendet.

4. Verfahren nach Anspruch 1, **dadurch gekennzeichnet, dass** man ein kontinuierliches chromatographisches Verfahren verwendet.

5. Verfahren nach Anspruch 4, **dadurch gekennzeichnet, dass** man nach der Simulated-Moving-Bed-(SMB)-Flüssigkeitschromatographiemethode verfährt.

6. Verfahren nach Anspruch 1, **dadurch gekennzeichnet, dass** man ein diskontinuierliches chromatographisches Verfahren verwendet.

7. Verfahren nach Anspruch 1, **dadurch gekennzeichnet, dass** man die linksdrehenden und rechtsdrehenden optischen Isomere druch Anwendung von an sich bekannten Verdampfungs- und Kristallisationsmethoden isoliert.

8. Verfahren nach Anspruch 1, **dadurch gekennzeichnet, dass** man die flüssigchromatographische Trennung bei einer Temperatur zwischen +10˚C und +40˚C durchführt.

## Revendications

1. Procédé de séparation des isomères optiques du composé hexahydro-4-(hydroxyméthyl)-5-hydroxy-2H-cyclopenta [b]furan-2-one de formule (I)

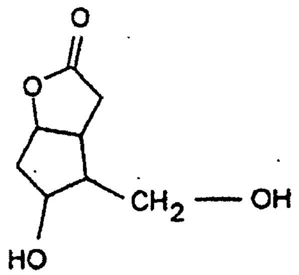

(I)

**caractérisé en ce que** le composé racémique de formule (I) ou le mélange des isomères optiques du composé de formule (I) dans un rapport de 1:1 est soumis à une résolution chromatographique en phase liquide où pendant ledit procédé sont appliqués comme phase stationnaire chirale de l'amylose-tris[(S)-α-méthylbenzylcarbamate] ou de l'amylose-tris-3,5-diméthylphénylcarbamate et comme phase mobile des mélanges méthanol/hexane normal ou des mélanges méthanol/acétonitrile ou des mélanges méthanol/alcool isopropylique ou des mélanges alcool isopropylique/n-hexane/méthanol ou des mélanges alcool isopropylique/n-hexane/éthanol ou du méthanol seul ou de l'acétonitrile seul, et si on le souhaite, les isomères optiques dextrogyre et lévogyre résultants sont isolés.

2. Procédé selon la revendication 1, **caractérisé en ce que** sont utilisés comme phase stationnaire chirale de l'amylose-tris-3,5-diméthylphénylcarbamate et comme phase mobile des mélanges d'acétonitrile et de méthanol.

3. Procédé selon la revendication 1, **caractérisé en ce que** sont utilisés comme phase stationnaire chirale de l'amylose-tris[(S)-α-méthylbenzylcarbamate] et comme phase mobile des mélanges alcool isopropylique/hexane normal ou des mélanges alcool isopropylique/hexane normal/méthanol.

4. Procédés selon la revendication 1, **caractérisé en ce qu'**un procédé chromatographique continu est employé.

5. Procédé selon la revendication 4, **caractérisé en ce qu'**un procédé chromatographique en phase liquide à lit mobile simulé (SMB) est employé.

6. Procédé selon la revendication 1, **caractérisé en ce qu'**un procédé chromatographique discontinu est employé.

7. Procédé selon la revendication 1, **caractérisé en ce que** les isomères optiques lévogyre et dextrogyre sont isolés en appliquant des procédés d'évaporation et de cristallisation connus en eux-mêmes.

8. Procédé selon la revendication 1, **caractérisé en ce que** la résolution chromatographique en phase liquide est effectuée à une température comprise entre +10 ˚C et +40 ˚C.

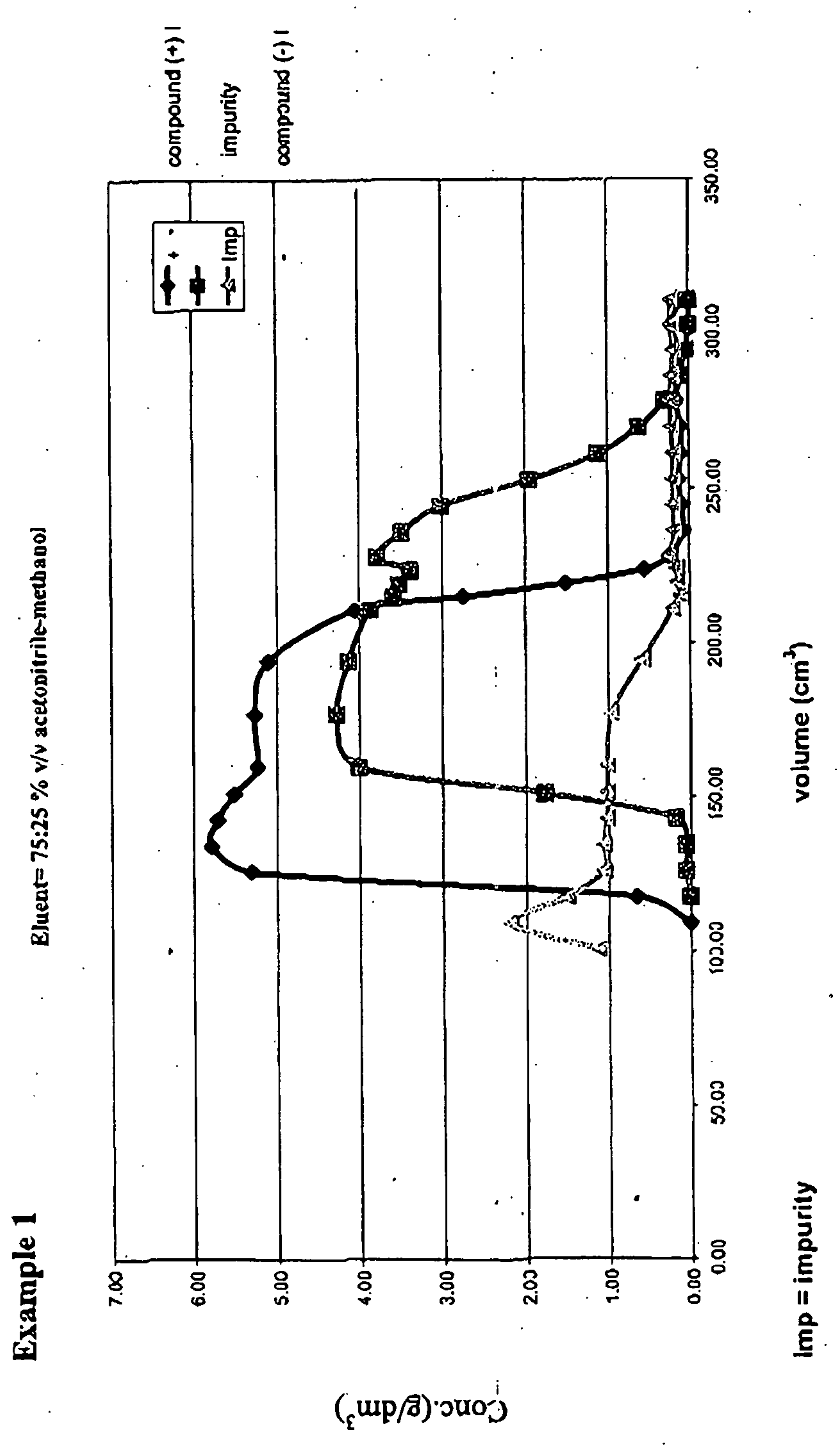
Example 1
Eluent= 75:25 % v/v acetonitrile-methanol
compound (+) I
impurity
compound (-) I
+
Imp
Conc.(g/dm3)
7.00
6.00
5.00
4.00
3.00
2.00
1.00
0.00
0.00
50.00
100.00
150.00
200.00
250.00
300.00
350.00
volume (cm3)
Imp = impurity

Figure 1

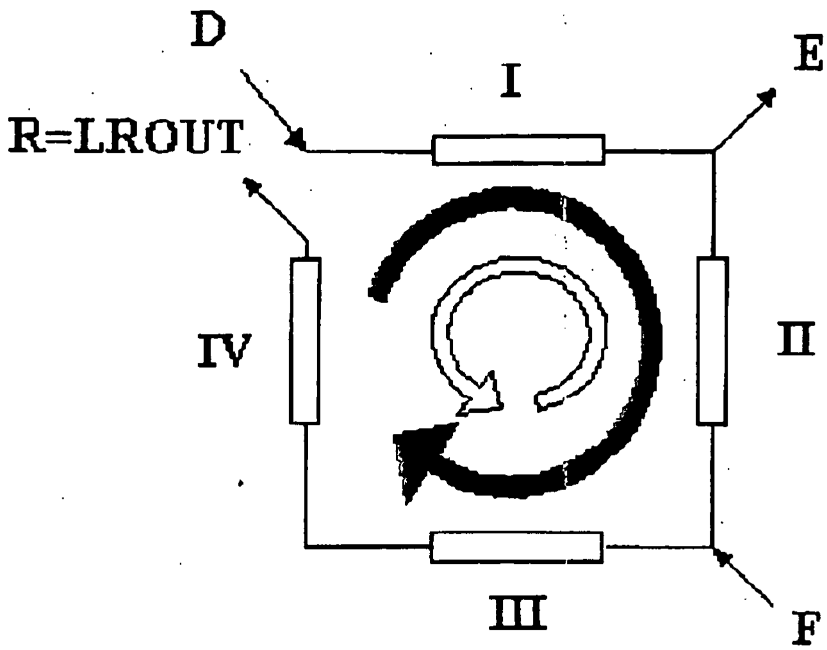


**Figure 2.** schematic drawing of the SMB equipment with 1:1:2:0 column configuration and open eluent circulation
White arrow: simulated moving direction of the solid phase
Black arrow: liquid phase flow direction.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 501310 A
- EP 719742 A2